# EUROPÄISCHE PATENTSCHRIFT

(11) **EP 1 118 309 B1**
(45) Veröffentlichungstag und Bekanntmachung des Hinweises auf die Patenterteilung: **01.10.2003**
(21) Anmeldenummer: 99111066.9
(22) Anmeldetag: 16.06.1999
(51) Int. Cl.: A61B 17/68

(54) **Interne Distraktionsvorrichtung**
Internal distraction device
Dispositif interne de distraction

(43) Veröffentlichungstag der Anmeldung: 25.07.2001
(73) Patentinhaber: Stryker Leibinger GmbH & Co. KG, 79111 Freiburg (DE)
(72) Erfinder: Donath, Raoul, 79639 Grenzach-Wyhlen (DE); Capanni, Felix, 79117 Freiburg (DE)
(74) Vertreter: von Hellfeld, Axel, Dr. Dipl.-Phys.

(56) Entgegenhaltungen:
- WO-A-96/19943
- WO-A-97/14367
- WO-A-99/04715
- US-A- 5 885 283

## Beschreibung

Die Erfindung betrifft eine interne Distraktionsvorrichtung zum Korrigieren von cranio-maxillo-fazialen Deformationen, die die im Oberbegriff des Patentanspruch 1 genannten Merkmale aufweist.

Mit einer derartigen Distraktionsvorrichtung ist es möglich eine Distraktion im cranio-maxillo-fazialen Bereich (CMF-Bereich), d.h. im Bereich von Schädel, Kiefer oder Gesicht durchzuführen. Ein Fachleuten bekanntes Beispiel dieser Distraktion ist die sogenannte LeFORT I und III Distraktion. Dabei wird eine chirurgisch erzeugte Fraktur an einem Knochen des CMF-Bereichs derart graduell und kontrolliert verlagert, daß sich sowohl das Weichteilgewebe als auch das Knochenvolumen simultan erweitern und eine Deformation des Knochens zurückgebildet (korrigiert) wird. Die Form des Knochens wird dadurch der natürlichen Knochenform angeglichen. Der Begriff "interne" Distraktionsvorrichtung bezeichnet hier ein Anordnen der Distraktionsvorrichtung unter der Haut (subkutan) oder in der Mundhöhle auf bzw. unter der Schleimhaut (supra- bzw. submukosal).

Für eine Anwendung im CMF-Bereich sind interne Distraktionsvorrichtungen bekannt, die eine lineare Korrektur eines Knochens ermöglichen. Bei einer linearen Korrektur besteht jedoch das Problem, das ein Winkelversatz des Knochens, der entweder als Deformation vorgegeben oder sich durch die Distraktion ergibt, nur schwer ausgeglichen werden kann. So bildet sich beispielsweise bei der Distraktion des Unterkieferkörpers ein sogenannter "offenen Biß", bei dem das Unterkiefer und das Oberkiefer bei geschlossener Stellung in einem Winkel von mehr als 10° zueinander geneigt sein können. Eine solche Neigung kann bis zu einem Winkel von 10° durch elastische Bänder ausgeglichen werden, die das Unterkiefer an das Oberkiefer fesseln. Bei einem Winkel von mehr als 10° ist jedoch eine Nachoperation erforderlich, um das Unterkiefer zum Oberkiefer rotatorisch zu korrigieren. Es ist zwar bekannt, einen Distraktionsvektor zu berechnen, der die erforderliche lineare und rotatorische Korrektur angibt, jedoch ist mit den bekannten internen Distraktionsvorrichtungen eine derartige Korrektur nicht möglich.

Bei derartigen internen Distraktionsvorrichtungen besteht ferner das Problem, daß mit ihnen jeweils nur eine Art von Befestigungsmittel, wie beispielsweise Knochenschrauben oder Maschinenschrauben, verwendet werden können. Dies ist besonders nachteilig, wenn der Knochen durch das Befestigungsmittel geschädigt worden ist und dieses daher gewechselt werden muß.

Darüber hinaus sind bei derartigen internen Distraktionsvorrichtungen Antriebswellen zum Verstellen der Distraktion sperrig angeordnet, so daß die Distraktionsvorrichtung beispielsweise in der Mundhöhle des Patienten umständlich zu plazieren, bei der Distraktion schwierig zu Handhaben und für den Patienten unangenehm zu tragen ist.

Für eine externe Anwendung, d.h. außen auf der Haut, sind Distraktionsvorrichtungen bekannt, mit denen eine mehrdimensionale Distraktion, also sowohl eine lineare als auch eine rotatorische Korrektur möglich ist. Die externe Anwendung einer Distraktionsvorrichtung bringt jedoch eine Vielzahl zusätzlicher Probleme mit sich. Die Distraktionsvorrichtung muß durch die Haut des Patienten geführt sein und ist daher vom Knochen entfernt angeordnet. Sie verformt sich bei der Distraktion, so daß bei dieser verhältnismäßig große Toleranzen auftreten. Die außerhalb der Haut angeordnete Distraktionsvorrichtung ist äußeren mechanischen Einwirkungen stärker ausgesetzt als eine interne Distraktionsvorrichtung, die durch Weichteile bedeckt ist. Ferner ist der Patient durch die von außen sichtbare Distraktionsvorrichtung psychisch stark belastet. Nach Entfernen der externen Distraktionsvorrichtung verbleiben an der Haut Narben, die zu kosmetischen Problemen führen können.

Eine interne Distraktionsvorrichtung mit einem an einem ersten Knochenfragment befestigbaren Lager und einem an einem zweiten Knochenfragment befestigbaren beweglichen Element ist jeweils aus der US 5,885,283 und der WO 99/04715 bekannt. Die beiden bekannten Distraktionsvorrichtungen sind jeweils derart ausgestaltet, dass sie neben einem Mechanismus zum linearen Bewegen des einen Knochenfragments relativ zum anderen Knochenfragment zusätzlich einen Mechanismus zum relativen Verschwenken der beiden Knochenfragmente umfassen.

Aus der WO 96/19943 ist eine Vorrichtung mit Mitteln zum Befestigen der Vorrichtung an einem Schädelknochen, Mitteln zum Halten von displazierten Knochensegmenten sowie Mitteln zum Einstellen der Lage der Knochensegmente relativ zum Schädelknochen bekannt. Zum Einstellen der Lage der Knochensegmente bezüglich des Schädelknochens sind eine Mehrzahl schwenkbarer Arme vorgesehen.

Die Aufgabe der Erfindung ist es eine interne Distraktionsvorrichtung vorzusehen, mittels der sowohl eine präzisere lineare Korrektur, im folgenden "Distraktion" genannt, als mit den oben erwähnten Vorrichtung erreichbar ist, als auch eine rotatorische Korrektur, im folgenden "Angulation" genannt, zum Korrigieren von cranio-maxillo-fazialen Deformationen möglich ist.

Die Aufgabe ist erfindungsgemäß durch eine interne Distraktionsvorrichtung mit den im Patentanspruch 1 genannten Merkmalen gelöst.

Demnach weist das Lager der Distraktionsvorrichtung eine Lagerplatte und eine Schwenkplatte auf, wobei die Schwenkplatte am Knochen befestigbar, an der Lagerplatte schwenkbar gelagert und relativ zu dieser schwenkbar ist. Durch die unmittelbar an der Lagerplatte abgestützte Schwenkplatte ist es möglich, die Angulation innerhalb enger Toleranzen auszuführen. Darüber hinaus ist die erfindungsgemäße Distraktionsvorrichtung nur unwesentlich größer als solche, die nur zur Distraktion geeignet sind.

Erfindungsgemäß ist der Schlitten auf zwei Führungswellen geführt, die zusammen mit der Lagerplatte und einem Gegenlager einen Distraktionsrahmen bilden. Somit ist eine besonders präzise Führung bei der Distraktion möglich.

Vorteilhaft weitergebildet ist die Erfindung, indem in der Schwenkplatte, dem Schlitten und/oder dem Gelenklager je mindestens eine Durchgangsöffnung zum Befestigen eines Knochenstiftes, einer Knochenschraube, einer Knochenplatte oder einer Maschinenschraube ausgebildet ist. Die bereitgestellte Durchgangsöffnung ermöglicht es verschiedenartige Befestigungsmittel zu befestigen, wobei die Durchgangsöffnung an die Maße bestimmter standardisierter Befestigungsmittel angepasst ist. Ferner können die Befestigungsmittel von beiden Seiten durch die Distraktionsvorrichtung geführt werden, so daß diese an beiden Seiten am Kopf des Patienten angesetzt werden kann. Die Durchgangsöffnung ist ferner an beiden Enden mit je einem Absatz versehen, in dem ein Kopf des Befestigungsmittels versenkbar ist. Die Distraktionsvorrichtung weist dadurch im angebrachten Zustand eine glatte äußere Oberfläche auf.

Bei einer vorteilhaften Weiterbildung ist im Schlitten eine Gewindebohrung ausgebildet, in der eine Distraktionsspindel eingeschraubt ist, die in der Lagerplatte drehbar gelagert und mittels einer Distraktionswelle drehbar ist. Durch die Distraktionsspindel kann der Schlitten besonders präzise eingestellt werden und ist in dieser Stellung selbsthemmend gehalten.

Die erfindungsgemäße Distraktionsvorrichtung ist vorteilhaft dadurch weitergebildet, daß in der Schwenkplatte eine Durchgangsöffnung ausgebildet ist, durch welche die Distraktionsspindel ragt, wobei diese insbesondere zwischen der Lagerplatte und der Schwenkplatte ein Kreuzgelenk umfaßt. Indem die Distraktionsspindel durch die Schwenkplatte geführt ist, ist es möglich, die Distraktion an dem Ende der Distrakionsvorrichtung anzutreiben, an dem die Schwenkplatte angeordnet ist. Dieses Ende ist bei der Distraktion in Richtung der Öffnung der Mundhöhle gerichtet. Die Distraktionsspindel kann dadurch, trotz der gleichzeitig möglichen Angulation, von außen zugänglich verstellt werden. Das an der Distraktionsspindel angeordnete Kreuzgelenk ermöglicht es, daß die Distraktionsspindel dem Schwenken der Schwenkplatte bei einer Angulation folgt.

Gemäß einer vorteilhaften Weiterbildung ist in der Schwenkplatte eine Gewindebohrung ausgebildet, in die eine Angulationsspindel eingeschraubt ist, die sich an der Lagerplatte abstützt. Durch die Angulationsspindel ist es möglich die Schwenkplatte gemäß der angestrebten Angulation an der Lagerplatte zu schwenken. Die Abstützung an der Lagerplatte ermöglicht eine präzise Führung der Schwenkbewegung. Insbesondere kann die Angulationsspindel an der Lagerplatte verschiebbar geführt sein, um eine Relativbewegung zwischen der Angulationsspindel und der Lagerplatte während der Angulation zu ermöglichen.

Bei einer vorteilhaften Ausgestaltung weist die Distraktionswelle und/oder die Angulationswelle je mindestens ein Kreuzgelenk auf. Alternativ sind/ist sie als je eine flexible Welle ausgeführt. Dadurch ist es möglich die Distraktionswelle und die Angulationswelle derart aus der Mundhöhle zu führen, daß sie für den Patienten keine zusätzliche Behinderung darstellen und dennoch von außen eine präzise Verstellung der Distraktion und der Angulation ermöglichen.

Das Kreuzgelenk oder die flexible Welle ist vorteilhaft mit einem flexiblen Schlauch überzogen, der verhindert, daß Weichteilgewebe oder Nahrungsmittel in die Zwischenräume des Kreuzgelenks oder der flexiblen Welle gelangen. Irritationen des Weichteilgewebes werden dadurch vermieden und die Hygiene verbessert.

Bei einer vorteilhaften Weiterbildung sind/ist die Distraktionswelle und/oder die Angulationswelle jeweils von der Distraktionsspindel und/oder der Angulationsspindel abnehmbar. Die Distraktions- und Angulationswelle können daher zum Anbringen der Distraktionsvorrichtung in der engen Mundhöhle abgenommen werden. Auch während einer Latenz-, einer Behandlung- und einer Konsolidierungsphase können die Wellen zeitweise entfernt werden, so daß die Distraktionsvorrichtung für den Patienten angenehmer zu tragen ist.

Vorteilhaft sind/ist die Distraktionswelle und/oder die Angulationswelle derart weitergebildet, daß sie je eine verschiedenartige Farbcodierung und/oder je einen verschiedenartigen Anschluß zum Ansetzen je eines Distraktionsschlüssels und/oder eines Angulationsschlüssels aufweisen. Die/der Schlüssel weisen selbst je eine entsprechende Farbcodierung und/oder je einen entsprechenden Anschluß auf. Damit ist eine direkte Zuordnung zwischen Schlüssel und Welle möglich. Verwechslungen und Falschbedienung der Distraktionsvorrichtung werden dadurch ausgeschlossen. Insbesondere können die Schlüssel mit einer Kennzeichnung der Drehrichtung versehen sein, so daß bei einer Verstellung von außen die nicht sichtbare Bewegung der internen Distraktionsvorrichtung nachvollziehbar ist.

Ein besonders bevorzugtes Ausführungsbeispiel der erfindungsgemäßen internen Distraktionsvorrichtung wird im folgenden anhand schematischer Zeichnungen erläutert. Es zeigt:
- Figur 1: eine erfindungsgemäße interne Distraktionsvorrichtung in einer Seitenansicht,
- Figur 2: die in Figur 1 dargestellte Distraktionsspindel und Distraktionswelle in einer Seitenansicht,
- Figur 3: die in Figur 1 dargestellte Lagerplatte in einer räumlichen Ansicht,
- Figur 4a: den in Figur 3 mit IVa-IVa bezeichneten Schnitt,
- Figur 4b: die in Figur 1 dargestellte Angulationsspindel in einer Seitenansicht,
- Figur 5: die in Figur 1 dargestellte Schwenkplatte in einer Seitenansicht,
- Figur 6: den in Figur 5 mit VI-VI bezeichneten Schnitt,
- Figur 7: einen erfindungsgemäßen Distraktionsschlüssel in einer Seitenansicht,
- Figur 8: die in Figur 7 mit IIX-IIX bezeichnete Ansicht,
- Figur 9: einen erfindungsgemäßen Angulationsschlüssel in einer Seitenansicht,
- Figur 10: die in Figur 9 mit X-X bezeichnete Ansicht, und
- Figur 11: einen erfindungsgemäßen Knochenstift in einer Seitenansicht.

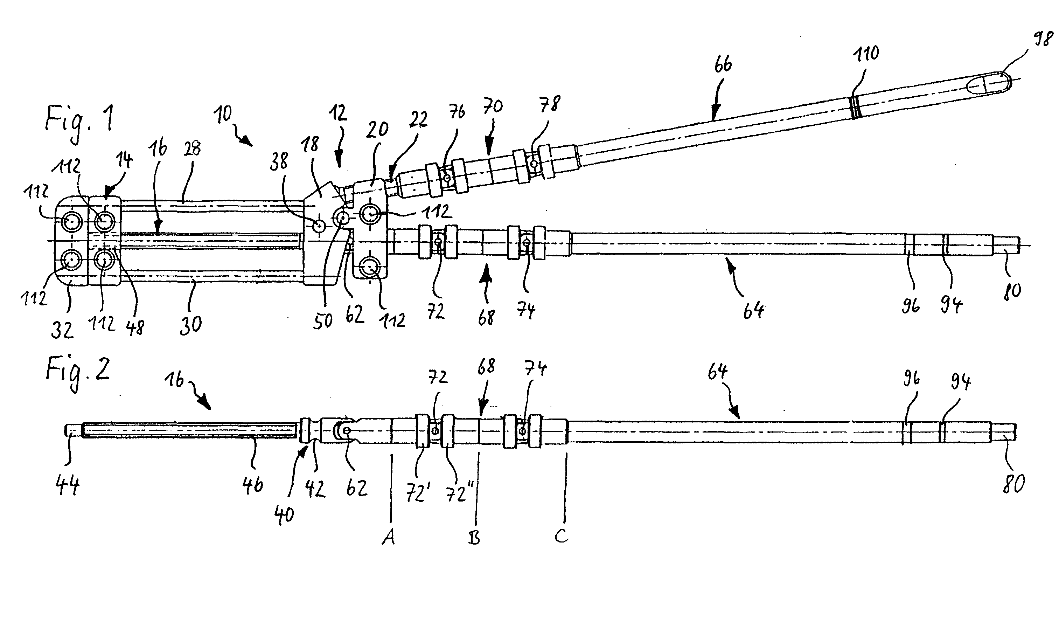

Figur 1 stellt eine Distraktionsvorrichtung 10 dar, die ein Lager 12 und einen Schlitten 14 aufweist, der relativ zum Lager 12 mittels einer Distraktionsspindel 16 verschiebbar ist. Beim chirurgischen Verfahren der Distraktion wird die Distraktionsvorrichtung 10 im cranio-maxillo-fazialen Bereich des Patienten an einem Knochen neben einer geplanten Fraktur mit dem Lager 12 und dem Schlitten 14 befestigt. Ein Anbringen der Distraktionsvorrichtung genau senkrecht zur geplanten Fraktur, wie bisher erforderlich, ist hierbei nicht notwendig, da ein Winkelversatz durch Angulation ausgeglichen werden kann. Anschließend wird der Knochen chirurgisch gebrochen. Mittels der Distrakionsspindel 16 wird der Schlitten 14 vom Lager 12 in bestimmten Schritten wegbewegt, dadurch die Fraktur aufgeweitet und die Bildung von Weichteilgewebe und Knochenvolumen in der Fraktur angeregt. Somit ist eine lineare Korrektur von Deformationen im CMF-Bereich möglich. Diese lineare Korrektur wird hier als "Distraktion" bezeichnet.

Neben der Distraktion soll auch eine rotatorische Korrektur eines Knochens ermöglicht werden, die hier als "Angulation" bezeichnet wird. Das Lager 12 weist dazu eine Lagerplatte 18 und eine daran schwenkbar angebrachte Schwenkplatte 20 auf. Die Schwenkplatte 20 ist am Knochen befestigbar und mittels einer Angulationsspindel 22 relativ zur Lagerplatte 18 und zum Schlitten 14 schwenkbar. Durch Verstellen der Distraktionsspindel 16 und der Angulationsspindel 22 kann während einer einzelnen Behandlung sowohl eine Distraktion als auch eine Angulation ausgeführt werden.

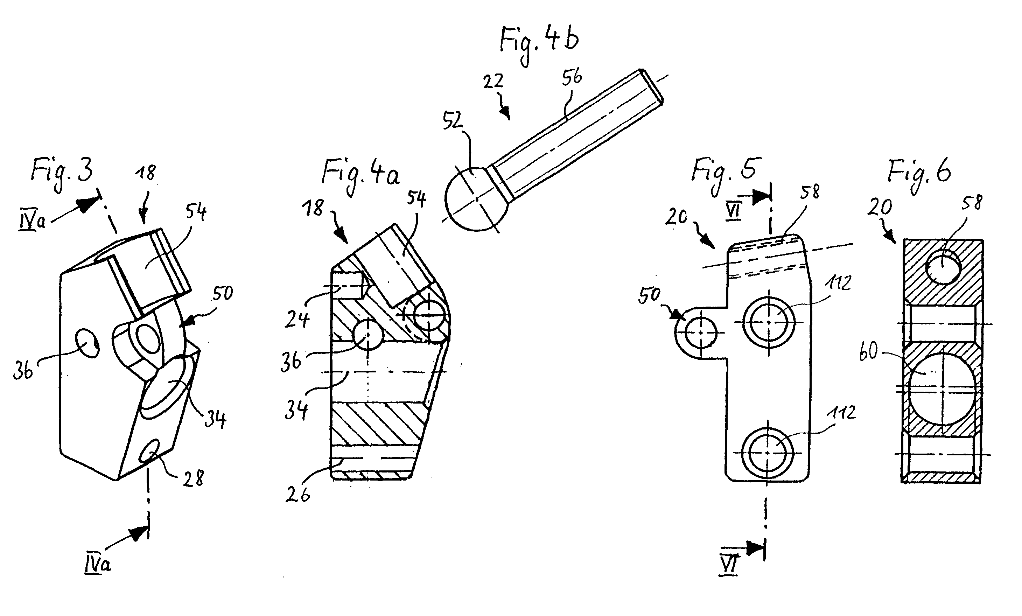

Mit Bezug auf die Figuren 2 bis 6 wird im Folgenden der Aufbau der Distraktionsvorrichtung 10 detailliert beschrieben.

Das Lager 12 weist in Richtung der Distraktionsspindel 16 zwei Bohrungen 24 und 26 auf, in die zwei Führungswellen 28 und 30 eingesetzt sind. Die Führungswellen 28 und 30 erstrekken sich parallel zur Distraktionsspindel 16 und sind mit ihrem der Lagerplatte 18 entgegengesetzten Ende an einem Gegenlager 32 angebracht. Die Lagerplatte 18 weist ferner eine in Richtung der Distraktionsspindel 16 verlaufende Durchgangsbohrung 34 auf, die eine Querbohrung 36 schneidet. In der Querbohrung 36 ist ein Stift 38 eingepreßt und verschweißt. Die Distraktionsspindel 16 durchsetzt die Durchgangsbohrung 34 und ist in dieser mittels eines Gleitlagers 40 geführt. Das Gleitlager 40 weist eine Umfangsnut 42 auf, in die sich der Stift 38 erstreckt. Die Distraktionsspindel 16 ist so an der Lagerplatte 18 nicht verschiebbar, sondern nur drehbar gelagert. Die Distraktionsspindel 16 ist am Gegenlager 32 durch ein zweites Gleitlager 44 drehbar gelagert. Zwischen den beiden Gleitlagern 40 und 44 ist an der Umfangsfläche ein Spindelgewinde 46 ausgebildet. Das Spindelgewinde 46 durchsetzt den Schlitten 14 in einer Gewindebohrung 48, so daß dieser durch Drehen der Distraktionsspindel 16 verschiebbar ist, wobei er auf den Führungswellen 28 und 30 gleitet und damit linear geführt ist.

Das Lager 12, die Führungswellen 28 und 30 und das Gegenlager 32 bilden zusammen einen Distraktionsrahmen. An diesem ist an der Lagerplatte 18 die Schwenkplatte 20 mittels eines Scharniergelenks 50 in der Ebene des Distraktionsrahmens schwenkbar gelagert.

Die Schwenkplatte 20 kann mittels der Angulationsspindel 22 verstellt werden. Hierzu weist die Angulationsspindel 22 an ihrem zur Lagerplatte 18 gerichteten Ende einen Kugelkopf 52 auf, der in eine zur Angulationsspindel 22 hin offene, kreiszylinderförmige Führungsbahn 54 greift, die in der Lagerplatte 18 quer zur Angulationsspindel 22 ausgenommen ist. Der Kugelkopf 52 der Angulationsspindel 22 ist in der Führungsbahn 54 der Lagerplatte 18 gehalten und dabei quer zur Angulationsspindel 22 verschiebbar. Die Angulationsspindel 22 weist ferner ein Spindelgewinde 56 auf, das sich an den Kugelkopf 52 anschließt und sich bis zu ihrem anderen Ende erstreckt. Das Spindelgewinde 56 ist in eine Gewindebohrung 58 eingeschraubt, welche die Schwenkplatte 20 durchsetzt.

Die Lagerplatte 18 und die Schwenkplatte 20 sind durch das Scharniergelenk 50 und die Angulationsspindel 22 relativ zueinander schwenkbar positioniert und nur durch Drehen der Angulationsspindel 22 verstellbar. Die Lage des Scharniergelenks 50 ist dabei von der in die Gewindebohrung 58 eingeschraubten Angulationsspindel 22 etwa ein Drittel der gesamten Länge der Schwenkplatte 20 entfernt. Durch diese verhältnismäßig kurze Distanz ergibt sich ein kurzer Hebelarm, so daß durch eine geringe Verstellung der Angulationsspindel 22 eine große Schwenkbewegung der Schwenkplatte 20 in einem Winkel von etwa 50° möglich ist. Das Scharniergelenk 50 kann auch weiter entfernt von der Angulationsspindel 22 angeordnet sein, wodurch eine zwar kleinere, dafür aber präzisere Schwenkbewegung der Schwenkplatte 20 ausgeführt werden kann.

In Fortsetzung der Durchgangsbohrung 34 der Lagerplatte 18 ist in der Schwenkplatte 20 eine langlochförmige Durchgangsöffnung 60 ausgebildet, durch die die Distraktionsspindel 16 ragt. Dabei weist die Distraktionsspindel 16 zwischen der Lagerplatte 18 und der Schwenkplatte 20 ein Kreuzgelenk 62 auf, so daß sie in diesem Bereich abknicken und der Schwenkbewegung der Schwenkplatte 20 folgen kann.

Um die Distraktionsspindel 16 beispielsweise von außerhalb der Mundhöhle des Patienten drehen zu können, ist auf dieser eine Distraktionswelle 64 angebracht. Analog weist die Angulationsspindel 22 eine Angulationswelle 66 auf. Die Distraktionswelle 64 und die Angulationswelle 66 weisen je eine Gelenkwelle 68 bzw. 70 mit zwei Kreuzgelenken 72 und 74 sowie 76 und 78 auf. die Kreuzgelenke 72 bis 78 haben einen Gelenkwinkel von ca. 40°. Dieser Gelenkwinkel ist durch am Kreuzgelenk 72 beispielhaft gekennzeichnete Ansätze 72', 72" begrenzt, so daß ein Verkanten und eine mechanische Beschädigung der Kreuzgelenke 72 bis 78 vermieden ist. Die Gelenke der Kreuzgelenke 72 bis 78 und des Kreuzgelenkes 62 sind mit im wesentlichen glatten Oberflächen und Bauteilübergängen ausgebildet, so daß beim Drehen der Kreuzgelenke 72 bis 78 Weichteilirritationen vermieden werden. Zusätzlich oder alternativ können die Kreuzgelenke 72 bis 78 in einem flexiblen Schlauch, wie beispielsweise einem Teflonschlauch, angeordnet sein. Ferner können die Distraktionswelle 64 und die Angulationswelle 66 als flexible Wellen ausgebildet und dabei ebenfalls mit einem flexiblen Schlauch überzogen sein.

Um die Distraktionswelle 64 und die Angulationswelle 66 abnehmen und aus der Mundhöhle des Patienten entfernen zu können, sind diese teilbar gestaltet, beispielsweise zusammensteckbar. Dies ist in Fig. 2 durch die Linien an den Bezugszeichen A, B und C dargestellt.

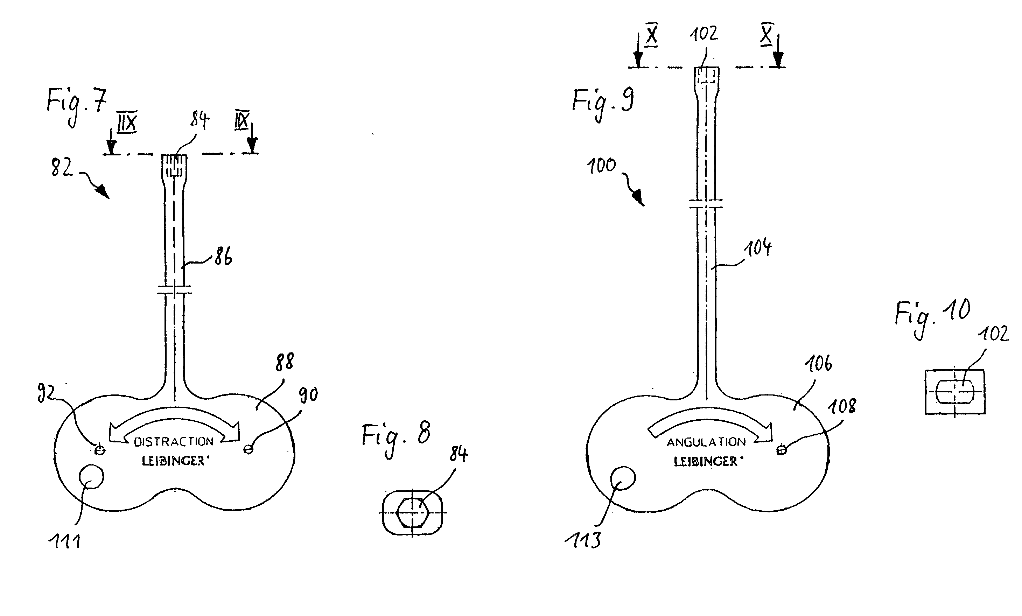

Die Distraktionswelle 64 weist an ihrem der Distraktionsspindel 16 gegenüberliegenden Endbereich einen sechskantförmigen Anschluß 80 auf, auf den ein Distraktionsschlüssel 82 aufsetzbar ist. Der in den Figuren 7 und 8 dargestellte Distraktionsschlüssel 82 weist hierfür eine sechskantförmige Aussparung 84 auf, die sich im Endbereich eines Schafts 86 befindet. Am entgegengesetzten Ende des Schafts 86 ist ein Griff 88 ausgebildet, auf dem Farbcodierungen 90 und 92 sowie eine Angabe der Drehrichtung des Distraktionsschlüssels 82 ausgebildet sind. Die Farben der Farbcodierungen 90 und 92 stimmen mit den Farben ringförmiger Farbcodierungen 94 und 96 an der Umfangsfläche der Distraktionswelle 64 überein.

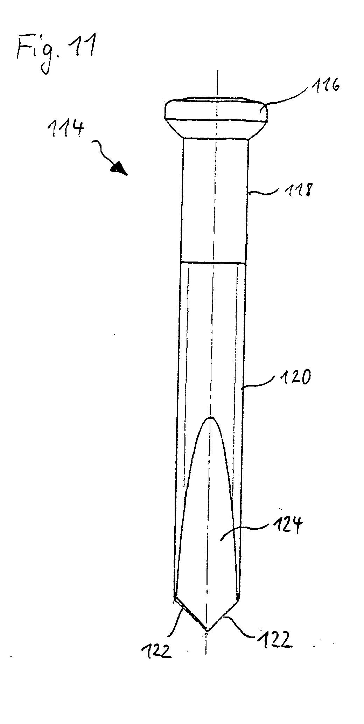

Die Angulationswelle 66 weist an ihrem der Angulationsspindel 22 gegenüberliegenden Endbereich einen beidseitig abgeflachten Anschluß 98 auf, der sich damit vom sechskantförmigen Anschluß 80 der Distraktionswelle 64 unterscheidet. Ein hierzu passender Angulationsschlüssel 100 ist in den Figuren 9 und 10 dargestellt. Er weist einen Anschluß 102 in Form einer schlitzförmigen oder quaderförmigen Aussparung auf, die im Endbereich eines Schafts 104 ausgebildet ist. Am entgegengesetzten Ende des Schafts 104 ist ein Griff 106 mit einer Farbcodierung 108 und einer Angabe der Drehrichtung des Angulationsschlüssels 100 ausgebildet. Die Farbe der Farbcodierung 108 stimmt mit der Farbe einer Farbcodierung 110 an der Umfangsfläche der Angulationswelle 66 überein.

Darüber hinaus ist auf je einer Seite der Griffe 88 und 106 jeweils eine Durchgangsöffnung 111 bzw. 113 ausgebildet, durch die beim Drehen des Schlüssels 82 bzw. 100 jede halbe Umdrehung nachvollziehbar ist. Dies ist insbesondere von Vorteil, da bei der Distraktion und Angulation das Verstellen der Distraktionsvorrichtung allgemein üblich pro Tag mit einer bestimmten Anzahl halber Umdrehungen vorgegeben wird.

Der Schlitten 14 und die Schwenkplatte 20 weisen je zwei Durchgangsbohrungen 112 auf, in die verschiedenartige Befestigungsmittel, wie beispielsweise ein in Figur 11 dargestellter Knochenstift 114, eine Knochenschraube oder eine Maschinenschraube zum Befestigen einer Knochenplatte einsetzbar ist. Die Befestigungsmittel können von beiden Seiten in die Durchgangsbohrungen 112 eingesetzt werden. Die Durchgangsbohrungen 112 weisen hierzu zusätzlich Absätze auf, in die sich beim Befestigen die Köpfe der Befestigungsmittel absenken. Dadurch weist die Distraktionsvorrichtung 10 eine im wesentlichen glatte Oberfläche auf, was besondere bei einer subkutanen Anwendung vorteilhaft ist. Bei Kombination der Distraktionsvorrichtung 10 mit beispielsweise einer Knochenplatte ist jedoch auch eine suprakutane Anwendung möglich.

Außer dem Schlitten 14 und der Schwenkplatte 20 sind auch im Gegenlager 32 Durchgangsbohrungen 112 ausgebildet. Durch diese kann die Distraktionsvorrichtung 10 auch zum sogenannten Knochentransport verwendet werden. Dabei werden das Gegenlager 32 und die Schwenkplatte 20 an jeweils einem Ufer einer chirurgischen Fraktur befestigt. Der Schlitten 14 wird an einem dazwischenliegenden Knochenabschnitt befestigt, der durch den Schlitten 14 von einem Frakturufer zum gegenüberliegenden Frakturufer bewegt wird, um beispielsweise entferntes Knochenvolumen zu ergänzen.

Der in Figur 11 dargestellte, im wesentlichen kreiszylinderförmige Knochenstift 114 ist zum Befestigen der Distraktionsvorrichtung 10 an einem Knochen vorgesehen. Er weist an einem Ende einen Kopf 116 mit einem darin ausgebildeten Innenvierkant (nicht dargestellt) zum Ansetzten eines Schraubendrehers, insbesondere eines Winkelschraubendrehers, auf. Unterhalb des Kopfes erstreckt sich ein kreiszylinderförmiger Schaftabschnitt 118, der beim Befestigen der Distraktionsvorrichtung 10 in einer der Durchgangsbohrungen 112 geführt ist. Der Durchmesser dieses Schaftabschnitts 118 ist dazu auf den Durchmesser der Durchgangsbohrungen 112 abgestimmt. Unter dem Schaftabschnitt erstreckt sich ein Gewindeabschnitt 120, der ein Schraubengewinde 120 aufweist. Das Schraubengewinde 120 kann zum Einschrauben in einen Knochen verwendet werden. Ferner weist der Knochenstift 114 an dem dem Kopf 116 entgegengesetzten Ende zwei Schneiden mit je einem Lanzenanschliff 122 und zwei an der Umfangsfläche ausgebildete Abflachungen 124 auf (nur eine Abflachung 124 dargestellt). Der Lanzenanschliff 122 weist einen Bohrfreischliff in einem Winkel von etwa 7° auf. Die Abflachungen 124 sind in einem Winkel von etwa 13° zur Achse des Knochenstiftes 114 geneigt. Durch eine derartige Schneidgeometrie bohrt sich der Knochenstift 118 beim Befestigen selbstschneident in den Knochen.

## Patentansprüche

1. Interne Distraktionsvorrichtung (10) zum Korrigieren von cranio-maxillo-fazialen Deformationen, mit einem Lager (12), das an einem Knochen befestigbar ist, sowie einem beweglichen Element (14), das am Knochen befestigbar und relativ zum Lager (12) geführt verschiebbar ist, wobei das Lager (12) ein Lagerelement (18) und eine Schwenkplatte (20) umfasst und wobei die Schwenkplatte (20) am Knochen befestigbar, am Lagerelement (18) schwenkbar gelagert und relativ zu diesem schwenkbar ist,
**dadurch gekennzeichnet, daß** das Lagerelement als eine Lagerplatte (18) und das bewegliche Element als ein Schlitten (14) ausgestaltet ist, wobei der Schlitten (14) auf zwei Führungswellen (26, 30) geführt ist, die zusammen mit der Lagerplatte (18) und einem Gegenlager (32) einen Distraktionsrahmen bilden.

2. Distraktionsvorrichtung nach Anspruch 1,
**dadurch gekennzeichnet, daß** in der Schwenkplatte (20), dem Schlitten (14) und/oder dem Gegenlager (32) mindestens eine Durchgangsöffnung (112) zum Befestigen eines Knochenstifts (114), einer Knochenschraube, einer Knochenplatte oder einer Maschinenschraube ausgebildet ist.

3. Distraktionsvorrichtung nach einem der Ansprüche 1 oder 2,
**dadurch gekennzeichnet, daß** im Schlitten (14) eine Gewindebohrung (48) ausgebildet ist, in der eine Distraktionsspindel (16) eingeschraubt ist, die in der Lagerplatte (18) drehbar gelagert und mittels einer Distraktionswelle (64) drehbar ist.

4. Distraktionsvorrichtung nach Anspruch 3,
**dadurch gekennzeichnet, daß** in der Schwenkplatte (20) eine Durchgangsöffnung (60) ausgebildet ist, durch welche die Distraktionsspindel (16) ragt, wobei diese insbesondere zwischen der Lagerplatte (18) und der Schwenkplatte (20) ein Kreuzgelenk (62) umfaßt.

5. Distraktionsvorrichtung nach einem der vorhergehenden Ansprüche,
**dadurch gekennzeichnet, daß** in der Schwenkplatte (20) eine Gewindebohrung (58) ausgebildet ist, in die eine Angulationsspindel (22) eingeschraubt ist, die sich an der Lagerplatte (18) abstützt und insbesondere an der Lagerplatte (18) verschiebbar geführt ist.

6. Distraktionsvorrichtung nach einem der Ansprüche 3 bis 5,
**dadurch gekennzeichnet, daß** die Distraktionswelle (64) und/oder eine Angulationswelle (66) mindestens ein Kreuzgelenk (72, 74; 76, 78) umfassen/umfaßt oder eine flexible Welle sind/ist.

7. Distraktionsvorrichtung nach Anspruch 6,
**dadurch gekennzeichnet, daß** das Kreuzgelenk (72, 74; 76, 78) oder die flexible Welle in einem flexiblen Schlauch angeordnet ist.

8. Distraktionsvorrichtung nach einem der Ansprüche 3 bis 7,
**dadurch gekennzeichnet, daß** die Distraktionswelle (64) und/oder die Angulationswelle (66) jeweils von der Distraktionsspindel (16) und/oder der Angulationsspindel (22) abnehmbar sind/ist.

9. Distraktionsvorrichtung nach einem der Ansprüche 3 bis 8,
**dadurch gekennzeichnet, daß** die Distraktionswelle (64) und/oder die Angulationswelle (66) eine verschiedenartige Farbcodierung (94, 96; 110) und/oder einen verschiedenartigen Anschluß (80; 98) zum Ansetzen eines Distraktionsschlüssels (82) und/oder eines Angulationsschlüssels (100) aufweisen, die/der selbst eine entsprechende Farbcodierung (90, 92; 108) und/oder einen entsprechenden Anschluß (84; 102) und insbesondere eine Kennzeichnung der Drehrichtung aufweisen/aufweist.

## Claims

1. An internal distraction device (10) for correcting cranio-maxillo-facial deformations with a bearing (12) which can be attached to a bone, as well as with a movable element (14) which can be attached at the bone and can be guided to move relative to the bearing (12), with the bearing (12) comprising a bearing element (18) and a swivel plate (20), and wherein the swivel plate (20) can be attached at the bone, is pivotably supported at the bearing element (18) and can be pivoted relative to same,
**characterised in that** the bearing element is configured as a bearing plate (18) and the movable element as a carriage (14), with the carriage (14) being guided on two guide shafts (26, 30) which, together with the bearing plate (18) and a counter bearing (32), form a distraction frame.

2. The distraction device according to Claim 1,
**characterised in that** at least one through-hole (112) is formed in the swivel plate (20), the carriage (14) and/or the counter bearing (32) for installing a bone pin (114), a bone screw, a bone plate, or a machine screw.

3. The distraction device according to one of Claims 1 or 2,
**characterised in that** a threaded hole (48) is formed in the carriage (14), into which a distraction spindle (16) is screwed which is rotatably supported in the bearing plate (18) and rotatable by means of a distraction shaft (64).

4. The distraction device according to Claim 3,
**characterised in that** a through-hole (60) is formed in the swivel plate (20), through which the distraction spindle (16) protrudes which comprises a universal joint in particular between the bearing plate (18) and the swivel plate (20).

5. The distraction device according to one of the previous claims,
**characterised in that** a threaded hole (58) is formed in the swivel plate (20), into which an angulation spindle (22) is screwed which bears against the bearing plate (18) and is slidably guided in particular at the bearing plate (18).

6. The distraction device according to one of Claims 3 to 5,
**characterised in that** the distraction shaft (64) and/or one angulation shaft (66) comprises or comprise, respectively, at least one universal joint (72, 74; 76, 78) or is/are a flexible shaft.

7. The distraction device according to Claim 6,
**characterised in that** the universal joint (72, 74; 76, 78) or the flexible shaft is arranged within a flexible hose.

8. The distraction device according to one of Claims 3 to 7,
**characterised in that** the distraction shaft (64) and/or the angulation shaft (66) each is/are removable from the distraction spindle (16) and/or the angulation spindle (22).

9. The distraction device according to one of Claims 3 to 8,
**characterised in that** the distraction shaft (64) and/or the angulation shaft (66) comprise a different colour coding (94, 96; 119) and/or a different connecting portion (80; 98) for the application of a distraction wrench (82) and/or an angulation wrench (100) which itself/themselves comprises/comprise a corresponding colour coding (90, 92; 108) and/or a corresponding socket (84; 102) and in particular a sense of rotation marking.

## Revendications

1. Dispositif interne de distraction (10) pour la correction des déformations cranio-maxillo-faclales, comportant un support (12) pouvant être fixé sur un os, ainsi qu'un élément mobile (14) pouvant être fixé sur l'os et monté déplaçable par rapport au support (12), le support (12) comprenant un élément d'appui (18) et une plaque pivotante (20), et la plaque pivotante (20) pouvant être fixée sur l'os, étant montée à pivotement sur l'élément d'appui (18) et pouvant pivoter par rapport à ce dernier,
**caractérisé en ce que** l'élément d'appui est conçu pour former une plaque d'appui (18) et l'élément mobile, un chariot (14), le chariot (14) se déplaçant sur deux arbres de guidage (28, 30), lesquels forment conjointement avec la plaque d'appui (18) et une butée (32) un cadre de distraction.

2. Dispositif de distraction selon la revendication 1,
**caractérisé en ce qu'**au moins un orifice (112) destiné à la fixation d'un clou (114) à os, d'une vis à os, d'une plaque à os ou d'une vis mécanique est ménagé dans la plaque pivotante (20), le chariot (14) et/ou la butée (32).

3. Dispositif de distraction selon l'une des revendications 1 ou 2,
**caractérisé en ce qu'**un alésage fileté (48) dans lequel est vissée une broche de distraction (16), laquelle est montée à rotation dans la plaque d'appui (18) et peut pivoter au moyen d'un arbre de distraction (64), est ménagé dans le chariot (14).

4. Dispositif de distraction selon la revendication 3,
**caractérisé en ce qu'**un orifice (60), au travers duquel la broche de distraction (16) fait saillie, est ménagé dans la plaque pivotante (20), la broche de distraction comprenant un joint universel (62) situé en particulier entre la plaque d'appui (18) et la plaque pivotante (20).

5. Dispositif de distraction selon l'une des revendications précédentes,
**caractérisé en ce qu'**un alésage fileté (58), dans lequel est vissée une broche d'angulation (22), laquelle est en appui sur la plaque d'appui (18) et montée déplaçable en particulier à la plaque d'appui (18), est manégé dans la plaque pivotante (20).

6. Dispositif de distraction selon l'une des revendications 3 à 5,
**caractérisé en ce que** l'arbre de distraction (64) et/ou un arbre d'angulation (66) comprennent/comprend au moins un joint universel (72, 74 ; 76, 78) ou constituent/constitue un arbre souple.

7. Dispositif de distraction selon la revendication 6,
**caractérisé en ce que** le joint universel (72, 74 ; 76, 78) ou l'arbre souple est disposé dans un flexible.

8. Dispositif de distraction selon l'une des revendications 3 à 7,
**caractérisé en ce que** l'arbre de distraction (64) et/ou l'arbre d'angulation (66) peuvent/peut respectivement être démonté(s) de la broche de distraction (16) et/ou de la broche d'angulation (22).

9. Dispositif de distraction selon l'une des revendications 3 à 8,
**caractérisé en ce que** l'arbre de distraction (64) et/ou l'arbre d'angulation (66) affichent un codage de couleur (94, 96 ; 110) de nature différente et/ou un raccord (80 ; 98) de nature différente pour l'introduction d'une clé de distraction (82) et/ou d'une clé d'angulation (100), lesquelles/laquelle présentent/présente elle(s)-même(s) un codage de couleur (90, 92 ; 108) correspondant et/ou un raccord (84 ; 102) correspondant et en particulier un repérage du sens de rotation.
